(19) Europäisches Patentamt
European Patent Office
Office européen des brevets

(11) Veröffentlichungsnummer : **0 445 641 B1**

(12) **EUROPÄISCHE PATENTSCHRIFT**

(45) Veröffentlichungstag der Patentschrift :
**05.01.94 Patentblatt 94/01**

(51) Int. Cl.[5] : **C07C 233/31,** C07C 231/12, C08F 220/58, C09J 133/26, C09J 7/02

(21) Anmeldenummer : **91102904.9**

(22) Anmeldetag : **28.02.91**

(54) **Ethylenisch ungesättigte Verbindungen.**

(30) Priorität : **08.03.90 DE 4007318**

(43) Veröffentlichungstag der Anmeldung :
**11.09.91 Patentblatt 91/37**

(45) Bekanntmachung des Hinweises auf die Patenterteilung :
**05.01.94 Patentblatt 94/01**

(84) Benannte Vertragsstaaten :
**AT BE CH DE ES FR GB IT LI NL SE**

(56) Entgegenhaltungen :
**DE-A- 3 820 464**

(56) Entgegenhaltungen :
**CHEMICAL ABSTRACTS, Band 88, Nr. 8, 20. Februar 1978, Columbus, Ohio, US; H. KAMOGAWA "Facile syntheses of 2,4-dihydroxybenzophenone derivatives with polymerizable vinyl groups", Seite 2, Zusammenfassung-Nr. 51 196t**
**PATENT ABSTRACTS OF JAPAN, unexamined applications, C Field, Band 12, Nr. 103, 5. April 1988, THE PATNT OFFICE JAPANESE GOVERNMENT, Seite 9 C 485**
**ENCYCLOPEDIA OF POLYMER SCIENCE AND ENGINEERING, vol. 15, 1989, S. 564-565, John Wiley & Sons**

(73) Patentinhaber : **BASF Aktiengesellschaft**
**Carl-Bosch-Strasse 38**
**D-67063 Ludwigshafen (DE)**

(72) Erfinder : **Rehmer, Gerd, Dr.**
**Koenigsbacher Strasse 1**
**W-6711 Beindersheim (DE)**
Erfinder : **Bott, Kaspar, Dr.**
**Werderstrasse 57**
**W-6800 Mannheim 1 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ethylenisch ungesättigte Verbindungen der allgemeinen Formel I

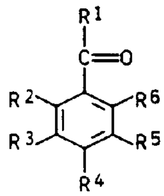

(I),

in der die Variablen folgende Bedeutung haben:

$R^1$ eine 1 bis 4 C-Atome enthaltende Alkylgruppe, einen Cyclopropyl-, -pentyl- oder -hexylrest, die Phenylgruppe oder eine Phenylgruppe, deren Wasserstoffatome teilweise oder vollständig durch Reste $R^4$ ersetzt sind, wobei nicht mehr als zwei Substituenten $R^4$ identisch sind, sowie $R^1$ gemeinsam mit $R^2$ oder gemeinsam mit $R^6$ eine $-CH_2-CH_2-$ oder $-CH_2-CH_2-CH_2-$Brücke,

$R^4$ eine 1 bis 24 C-Atome enthaltende Alkylgruppe,

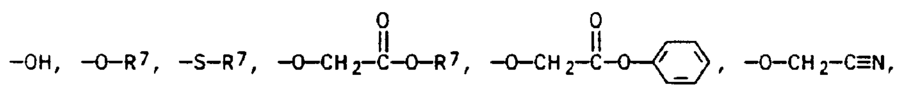

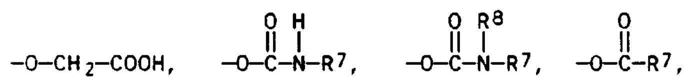

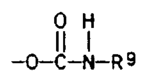 oder 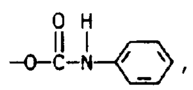 ,

$R^2$ oder $R^6$ unabhängig voneinander Wasserstoff, einen der Reste $R^4$ mit der Ausnahme der OH-Gruppe, oder für den Fall, daß $R^1$ eine Arylgruppe ist, eine direkte Bindung an $R^1$ in ortho-Stellung zur Carbonylgruppe, und

$R^{3,}$ $R^5$ Wasserstoff, einen der Reste $R^4$ oder eine Gruppe der allgemeinen Formel II

;

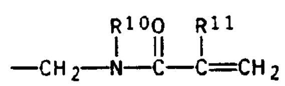

(II),

wobei

$R^7$, $R^8$ eine 1 bis 4 C-Atome enthaltende Alkylgruppe,

$R^9$ eine 5 oder 6 C-Atome enthaltende Cycloalkylgruppe,

$R^{10}$ Wasserstoff oder eine 1 bis 4 C-Atome enthaltende Alkylgruppe und

$R^{11}$ Wasserstoff oder eine 1 bis 4 C-Atome enthaltende Alkylgruppe bedeuten,

mit der Maßgabe, daß entweder $R^3$ oder $R^5$ eine Gruppe der allgemeinen Formel II ist.

Weiterhin betrifft die vorliegende Erfindung die Herstellung von Verbindungen I sowie deren Verwendung als copolymerisierbare Monomere, insbesondere zur Herstellung von Polymerisaten, die nach Einwirkung von aktinischer Strahlung eine erhöhte innere Festigkeit aufweisen.

Ethylenisch ungesättigte Verbindungen des allgemeinen Aufbaus

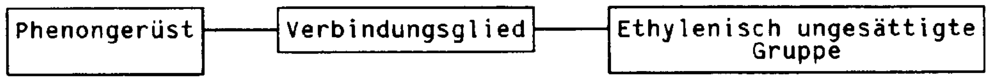


sind bekannt. Sie sollen nachfolgend als ethylenisch ungesättigte Phenone bezeichnet werden.

So enthält die US 3 214 492 Verbindungen der allgemeinen Formel III

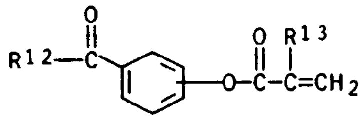 (III),

in der

$R^{12}$ $-CH_3$ oder $-C_6H_5$

und

$R^{13}$ -H oder $-CH_3$

bedeuten.

Ähnliche Acryloxy- oder Methacryloxygruppen enthaltende Acetophenon- oder Benzophenonderivate sind aus der US 3 429 852 bekannt.

Die DE-A 28 18 763 betrifft Verbindungen der allgemeinen Formel IV

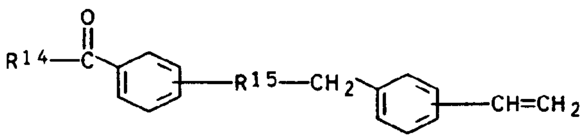 (IV),

in der

$R^{14}$ $-C_nH_{2n+1}$ mit n = 1 bis 3 oder $-C_6H_5$,

$R^{15}$ 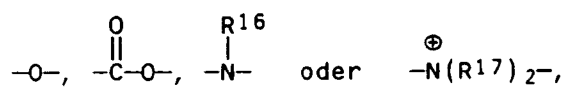 oder

$R^{16}$ -H oder $-C_nH_{2n+1}$ mit n = 1 bis 8

und

$R^{17}$ $-C_nH_{2n+1}$ mit n = 1 bis 4

bedeuten.

Die DE-A 38 20 464 beschreibt unter anderem die Verbindungen

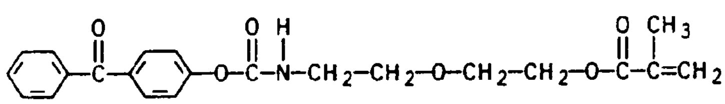

sowie

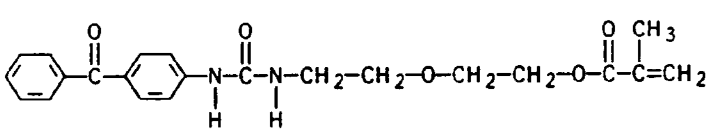

und DE-A 38 44 445 betrifft ethylenisch ungesättigte Acetophenon- und Benzophenonderivate, die als Verbindungsglied zwischen Phenongerüst und ethylenisch ungesättigter Gruppe eine Carbonatgruppe aufweisen.

Aus Chemical Abstracts, Bd. 88, Nr. 8, 20. Februar 1978, Columbus, Ohio, USA, Seite 2, Zusammenfassung Nr. 51 196t & J.Polym. Sci. Polym. Lett., Ed. 1977, 15 (11) 675-7 sind copolymerisierbare UV-Stabilisatoren durch Umsetzung von N-(Hydroxymethyl)acrylamid mit Benzophenonderivaten bekannt.

Die genannten Verbindungen werden in den verschiedenen Schriften als copolymerisierbare Monomere zur Herstellung von Polymerisaten, die nach Einwirkung von aktinischer Strahlung eine erhöhte innere Festigkeit aufweisen, empfohlen. Nachteilig an diesen bekannten ethylenisch ungesättigten Phenonen ist jedoch, daß sie nicht gleichzeitig die nachfolgenden Eigenschaften aufweisen:

a) erhöhte photochemische Reaktivität,

b) erhöhte Hydrolysebeständigkeit der Verbindung von ethylenisch ungesättigter Gruppe und Phenongerüst,

c) erhöhte thermische Stabilität der Verbindung von ethylenisch ungesättigter Gruppe und Phenongerüst,

d) erhöhte Löslichkeit in den Comonomeren,

e) in einfacher Weise herzustellen,

f) in einfacher Weise zu reinigen und

g) gut radikalisch copolymerisierbar.

Ziel der vorliegenden Erfindung war es daher, ethylenisch ungesättigte Phenone zur Verfügung zu stellen, die sich insbesondere als copolymerisierbare Monomere zur Herstellung von Polymerisaten, die nach Einwirkung von aktinischer Strahlung eine erhöhte innere Festigkeit aufweisen, eignen und in voll befriedigender Weise gleichzeitig die Eigenschaften a) bis g) aufweisen. Demgemäß wurden die eingangs definierten Verbindungen gefunden.

Besonders geeignete Verbindungen I sind solche, die als $R^4$ eine 1 bis 4 C-Atome aufweisende Alkoxygruppe, vorzugsweise die Methoxy- oder die Ethoxygruppe, enthalten, wobei diejenigen bevorzugt werden, die darüber hinaus als $R^{11}$ Wasserstoff oder die Methylgruppe, als $R^1$ die Phenylgruppe sowie als $R^{10}$, $R^2$, $R^3$ und $R^6$ jeweils Wasserstoff aufweisen.

Die erfindungsgemäßen Phenone sind in der Regel in einfacher Weise erhältlich durch Umsetzen einer Verbindung der allgemeinen Formel V

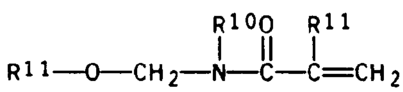

(V)

mit einer Verbindung der allgemeinen Formel VI

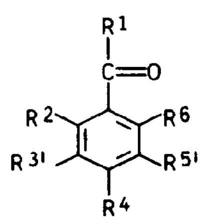

(VI),

in der einer der Reste $R^{3'}$ oder $R^{5'}$ Wasserstoff und der andere ein Rest $R^4$ ist.

Die Umsetzung folgt dem allgemeinen Reaktionsschema VII

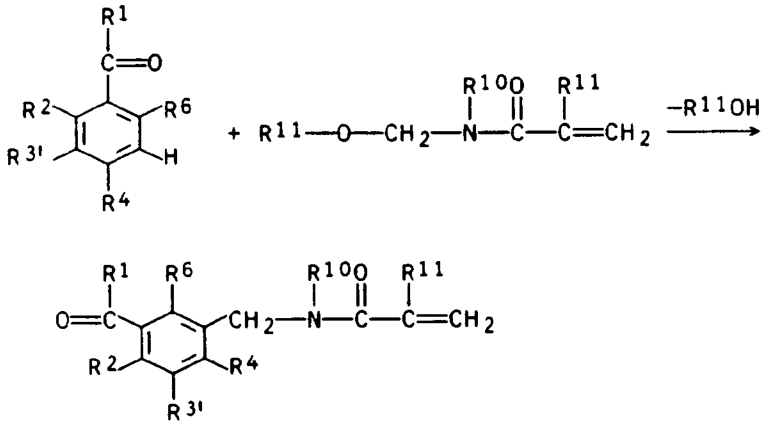

(VII)

Dieser Reaktionstyp ist als "Amidomethylierung" in der Literatur bekannt und die zugehörigen Reaktionsbedingungen sind u.a. in den folgenden Quellen vorbeschrieben:

- H. Hellmann, G. Aichinger und P. Wiedemann, J. Liebigs Ann. Chemie 626, S. 35 (1959)
- H. E. Zaug, W.B. Martin, Organic Reactions 14, S. 52 (1965)
- H. E. Zaug, Synthesis S. 49 (1970)

In der Regel werden die Verbindungen (V) und (VI) in äquimolaren Mengen eingesetzt. Die Reaktionstemperatur beträgt normalerweise -10 bis 100°C. Vorzugsweise wird bei 0 bis 70°C und besonders bevorzugt bei 5 bis 30°C gearbeitet. Üblicherweise wird die Umsetzung säurekatalysiert durchgeführt. Diesbezüglich besonders geeignete Protonensäuren sind Ameisensäure, Essigsäure, Phosphorsäure, Propansulfonsäure, Ethansulfonsäure, Methansulfonsäure sowie besonders bevorzugt Schwefelsäure-Wasser-Gemische. Die Umsetzung kann in Gegenwart oder in Abwesenheit eines Lösungsmittels durchgeführt werden. Als Lösungsmittel eignen sich alle säurebeständigen, bezüglich einer Amidoalkylierung inerten, unter den Reaktionsbedingungen flüssigen sowie die Reaktionspartner lösenden Mittel, wie Methylenchlorid oder Acetonitril. Vorzugsweise erfolgt die Umsetzung in Abwesenheit von Lösungsmitteln. Um eine radikalische Polymerisation der ungesättigten Edukte oder Produkte zu unterbinden, wird die Umsetzung zweckmäßigerweise in Anwesenheit von Polymerisationsinhibitoren durchgeführt.

Als solche kommen z.B. Luftsauerstoff, Nitrobenzol, p-Nitroso-dimethylanilin, Chinon, Hydrochinon, Hydrochinonmonomethylether, 2,2-Diphenyl-1-pikrylhydrazyl, 2,6-Di-tert.-butyl-p-kresol, Phenothiazin oder Cu(II)-Salze wie Cu(II)-acetat oder Cu(II)-sulfat in Betracht. Die Reaktionsdauer beträgt in der Regel 1 bis 10 h. Nach beendeter Umsetzung wird das Reaktionsgemisch durch Versetzen mit Wasser, Eis oder Eis-Wasser-Gemischen der Hydrolyse unterworfen und nach an sich bekannten Verfahren, z.B. durch Extraktion mit organischen Lösungsmitteln wie Methylenchlorid oder Diethylether, gegebenenfalls nach vorübergehender Neutralisation, und anschließendes Entfernen des organischen Lösungsmittels, aufgearbeitet, um das Zielprodukt zu isolieren.

In günstigen Fällen scheidet sich das Zielprodukt bei der Hydrolyse unmittelbar fest ab und kann durch Filtration abgetrennt werden. In ungünstigen Fällen sind über die bereits genannten Maßnahmen hinaus zusätzliche Schritte wie Umkristallisieren, Destillieren, Sublimieren oder Zonenschmelzen für eine Reinisolierung erforderlich. Gegebenenfalls muß auch die organische Lösung der Reaktionsprodukte einer chromatographischen Trennung unterworfen werden. In der Regel erfolgt die Umsetzung jedoch in guter Selektivität.

Die Ausgangsverbindungen V und VI sind nach an sich bekannten Verfahren erhältlich oder im Handel käuflich zu erwerben. Gemäß H. Feuer und U. E. Lynch, J. Am. Chem. Soc. 75, S. 5027 (1953) und nach der US-3 064 050 erhält man z.B. N-(Hydroxymethyl)methacrylamid durch Umsetzen von Methacrylamid mit Paraformaldehyd in wasserfreiem Tetrachlorkohlenstoff.

N-(Hydroxymethyl)acrylamid ist durch analoge Umsetzung in wasserfreiem Ethylenchlorid erhältlich. In beiden Fällen beträgt die Ausbeute im allgemeinen 70 % d. Theorie. Die ungesättigten Carbonamid-N-methylalkyl-ether sind z.B. nach E. Müller, K. Dinges und W. Graulich, Makromol. Chem. 57 S. 27 (1962) durch Einwirkung von Alkanol auf die entsprechende Methylolverbindung erhältlich.

Von der Verbindungen VI ist z.B. 4,4'-Dihydroxybenzophenon bei der Fa. Riedel-de Haen, D-3016 Seelze 1 und 4-Methoxybenzophenon, 4-Methylbenzophenon bei der Fa. Aldrich-Chemie, D-7924 Steinheim erhältlich.

4-Hydroxybenzophenon ist in ca. 90%iger Ausbeute (d. Theorie) durch Friedel-Crafts-Acylierung von Phenol mit Benzoylchlorid in Nitrobenzol in Gegenwart von Aluminiumchlorid oder Titantetrachlorid erhältlich (Houben-Weyl 7/2a, S. 186, 1973). Eine isomerenfreie Herstellung derselben Verbindung ermöglicht die Oxidation von 4-Hydroxy-phenylmethan mit 5,6-Dichlor-2,3-dicyan-p-benzochinon (Houben-Weyl, 7/2a, S. 681, 1973). p-Methoxyacetophenon ist durch Friedel-Crafts-Acylierung von Anisol mit Acetylchlorid in wasserfreiem 1,2-Dichlorethan in Gegenwart von Aluminiumchlorid in ca. 60%iger Ausbeute (d. Theorie) erhältlich 0.(Organikum, 2. ber. Nachdruck der 15. Auflage, VEB Verlag der Wissenschaften, Berlin 1981).

Besonders geeignete Ausgangsverbindungen VI sind 4-Methoxybenzophenon, 4-Hydroxybenzophenon, 4-Methylbenzophenon, p-Methoxyacetophenon sowie p-Methylpropiophenon. Sie sind durchweg im Handel erhältlich.

Eine Variante zur Herstellung von Verbindungen I besteht darin, daß man von einer einfachen Verbindung VI ausgeht, diese einer Umsetzung gemäß dem allgemeinen Reaktionsschema VII unterwirft und das dabei erhältliche Zielprodukt durch Reaktion von im Zielprodukt enthaltenen funktionellen Gruppen nach gängigen Verfahren, z.B. durch Addition von Isocyanaten wie Phenylisocyanat oder Cyclohexylisocyanat an -OH, durch Acylierung oder durch Alkylierung derivatisiert. In diesem Zusammenhang sind solche Verbindungen VI als Ausgangsverbindungen von besonderem Interesse, in denen wenigstens einer der Substituenten $R^4$, $R^6$ die Hydroxylgruppe ist.

Die erfindungsgemäßen Phenone weisen in der Regel ein voll befriedigendes Kristallisationsverhalten auf und sind bei Zimmertemperatur üblicherweise fest, so daß sie durch Umkristallisieren leicht in hoher Reinheit erhältich sind. Sie eignen sich insbesondere als copolymerisierbare Monomere zur Herstellung von Polymerisaten, die nach Einwirkung von aktinischer Strahlung eine erhöhte innere Festigkeit aufweisen. Sie können jedoch auch mit sich selbst polymerisiert werden. Bemerkenswerterweise weisen sie insbesondere im kurz- bis längerwelligen UV-Bereich, 250 bis 400 nm, eine erhöhte photochemische Reaktivität auf.

Bei der Verwendung der erfindungsgemäßen Phenone zur Herstellung von Homo- oder Copolymerisaten können die üblichen Verfahren der Vinylpolymerisation in an sich bekannter Weise benutzt werden. Beispielsweise können solche Polymerisate durch radikalische Polymerisation in Masse, Suspension, Lösung oder Emulsion erhalten werden. Die Polymersiation der ethylenisch ungesättigten Phenone I kann aber auch durch ionische Katalystoren oder durch stereospezifische Katalysatoren vom Ziegler-Typ angeregt werden.

Vorzugsweise erfolgt die Polymerisation radikalisch initiiert. Dabei werden in der Regel Produkte erhalten, deren Restgehalt an ethylenisch ungesättigten Phenonen, bei einem Ausgangsanteil von 10 Gew.-%, weniger als 100 ppm beträgt (beide Angaben beziehen sich auf die Gesamtmenge der Ausgangsmonomeren). Die Bindung des Phenongerüsts an das Polymerrückgrat der so erhältlichen Polymerisate weist eine erhöhte thermische Stabilität sowie eine erhöhte Hydrolysebeständigkeit aus, weshalb die Polymerisate auch bei erhöhten Temperaturen in befriedigender Weise verarbeitet werden können und die Phenone I insbesondere für radikalisch initiierte wäßrige Emulsionspolymerisationsverfahren geeignet sind.

Geeignete Comonomere zu den Phenonen I sind beispielsweise Styrol, $\alpha$-Methylstyrol, Acrylsäure- und Methacrylsäureester von 1 bis 24 C-Atome enthaltenden Alkanolen wie Methylacrylat, Methylmethacrylat, Ethylacrylat, Ethylmethacrylat, n-Propylacrylat, n-Propylmethacrylat, n-, tert.- und iso-Butylacrylat und -methacrylat, 2-Ethylhexylacrylat und -methacrylat, iso-Amylacrylat, n-Heptylacrylat, iso-Octylacrylat, Isobornylmethacrylat und Isobornylacrylat, des weiteren Vinylester aliphatischer Carbonsäuren, die 2 bis 18 C-Atome enthalten, wie z.B. Vinylacetat oder Vinylpropionat, sowie Acrylamid, Methacrylamid, N-Vinylpyrrolidon, Vinylimidazol, N-Vinylformamid, N-Vinylcaprolactam, Acrylsäure, Methacrylsäure, Acrylnitril, Methacrylnitril, Maleinsäureanhydrid, Itaconsäureanhydrid, Maleinsäure und Fumarsäure, Diester, Diamide und Imide olefinisch ungesättigter Dicarbonsäuren wie z.B. Maleinsäurediimid, Dimethylmaleinat, Dimethylfumarat, Di-n-butylmaleinat oder Di-n-butylfumarat, sowie die Halbester und Halbamide olefinisch ungesättigter Dicarbonsäuren wie Mono-n-butylmaleinat und Mono-n-butylmaleinsäureamid, aber auch Monomere wie Vinylchlorid, Vinylidenchlorid, Vinylfluorid, Ethen, Propen, Butadien, Diallylphthalat und Isopren sowie Gemische der genannten Monomeren. Vorzugsweise werden monoethylenisch ungesättigte Verbindungen als Comonomere eingesetzt.

Die erfindungsgemäßen Phenone I sind in den genannten Monomeren normalerweise in voll befriedigender Weise löslich. In der Regel beträgt die Löslichkeit bei Normalbedingungen wenigstens 1 g Phenon I pro 100 g Comonomere.

Der Gewichtsanteil der Phenone I an den Copolymerisaten beträgt je nach Bedarf 0,01 bis 50, bevorzugt 0,1 bis 10 Gew.- %, bezogen auf das Copolymerisat.

Insbesondere eignen sich die Phenone I auch zur Herstellung von Propfcopolymerisaten, wobei das Phenon I in Gegenwart von zuvor hergestellten Vinylpolymeren wie Polyolefinen, Polyvinylhalogeniden oder Polyvinylestern polymerisiert wird.

Unabhängig vom Polymerisationsverfahren, nach dem die die Phenone I einpolymerisiert enthaltenden Polymerisate hergestellt werden, zeigen diese stets eine Empfindlichkeit gegenüber aktinischer Strahlung, insbesondere im Wellenlängenbereich von 250 bis 400 nm, und weisen nach deren Einwirkung eine erhöhte innere Festigkeit auf. Diese erhöhte innere Festigkeit weist sich z.B. durch eine erhöhte Steifigkeit, einen erhöhten Schmelzpunkt sowie eine reduzierte Löslichkeit des Polymerisats in den verschiedensten Lösungsmitteln und einer daraus resultierenden erhöhten Beständigkeit gegenüber Ölen, Fetten, Wasser und dergleichen aus. Diese Eigenschaften sind vielfach erwünscht, so z.B. bei der Verwendung von Polymerisaten bei Photoreproduktionsverfahren. Aber auch bei der Verwendung solcher Polymerisate zum Beschichten oder Imprägnieren sind diese Eigenschaften von Vorteil. Üblicherweise wird das die Phenone I einpolymerisiert enthaltende Polymerisat erst nach seiner Verformung zu einem Film, einem Überzug oder einem anderen Gegenstand, aktinischer Strahlung ausgesetzt.

Insbesondere eignen sich die erfindungsgemäßen Phenone zur Herstellung von Polymerisaten, die nach ultravioletter Bestrahlung als Haftklebestoffe mit erhöhter Scherfestigkeit unter statischer Belastung geeignet sind. Außerdem zeigen diese Haftklebstoffe eine erhöhte Schälfestigkeit. Vorzugsweise sind sie in polymerisierter Form aufgebaut aus

a) 0,25 bis 5 Gew.-% wenigstens eines Phenons I und

b) 95 bis 99,75 Gew.-% wenigstens eines copolymerisierbaren monoethylenisch ungesättigten Monomeren.

Von besonderem Interesse für Haftkleber sind Copolymerisate, deren Monomerenzusammensetzung so bemessen ist, daß ein nur aus den Monomeren b) aufgebautes Polymerisat eine Glasübergangstemperatur von -45 bis 0°C, besonders bevorzugt von -30 bis -10°C, aufweisen würde. Nach Fox (T.G.Fox, Bull. Am. Phys. Soc. (Ser. II) 1,123 [1956]) gilt für die Glasübergangstemperatur von Mischpolymerisaten in guter Näherung:

$$\frac{1}{T_g} = \frac{X^1}{T_g^1} + \frac{X^2}{T_g^2} + \ldots\ldots\frac{X^s}{T_g^s}$$

wobei $X^1$, $X^2$, ....., $X^s$ die Massenbrüche der Monomeren, 1, 2, ....., s und $T_g^1$, $T_g^2$, .... $T_g^s$ die Glasübergangstemperaturen der jeweils nur aus einem der Monomeren 1, 2, .... oder s aufgebauten Polymeren in Grad Kelvin bedeuten. Die Glasübergangstemperaturen von obengenannten Monomeren b) sind im wesentlichen bekannt und z.B. in J. Brandrup, E. H. Immergut, Polymer Handbook 1$^{St}$ Ed. J. Wiley, New York 1966 und 2$^{nd}$ Ed. J. Wiley, New York 1975 aufgeführt.

Darüber hinaus weisen als solche Haftklebstoffe geeignete Polymerisate vor iher Bestrahlung in Tetrahydrofuran (THF) bei 25°C vorzugsweise einen K-Wert von 20 bis 70, besonders bevorzugt von 30 bis 55 auf.

Der K-Wert ist eine relative Viskositätszahl, die in Analogie zur DIN 53 726 bestimmt wird. Er enthält die Fließgeschwindigkeit einer 1 gew.-%igen Lösung des Polymerisats in THF, relativ zur Fließgeschwindigkeit von reinem THF und charakterisiert das mittlere Molekulargewicht des Polymerisats. Die Anfangsoberflächenklebrigkeit der beschriebenen Haftklebstoffe kann man modifizieren, indem man den erfindungsgemäßen Polymerisaten in Mengen von bis zu 50 Gew.-% klebrigmachende Harze (Tackifier) wie Cumaron-Inden-, Alkylphenol-Formaldehyd- oder Alkydharze zugibt. In untergeordneten Mengen können den als Haftklebstoffen geeigneten erfindungsgemäßen Polymerisaten vor ihrer Anwendung auch mineralische Füllstoffe, Weichmacher, polychlorierte Kohlenwasserstoffe oder Paraffinöle zugesetzt werden.

Anwendungstechnisch bevorzugt ist die Verwendung der erfindungsgemäßen Haftklebstoffe zur Herstellung selbstklebender Artikel, insbesondere zur Herstellung von Klebebändern und -folien, die ganz allgemein aus einem Trägermaterial und einem Haftklebstoff bestehen. Je nach Anwendungsfall werden die Trägermaterialien aus unterschiedlichen Substraten ausgewählt. Geeignet sind u.a. textile Gewebe, Papiere, Kunststoff-Folien aus Polyvinylchlorid, Polyester wie Polyethylenglycolterephthalat, Celluloseacetat, Polypropylen, Metallfolien aus Aluminium, Kupfer oder Blei, aber auch Schaumstoffe aus Polyurethan, Polyvinylchlorid, Polyethylen sowie Polychloropren. Die Applikation der erfindungsgemäßen Haftklebstoffe erfolgt bevorzugt vor der Bestrahlung mit ultraviolettem Licht. Sie kann aus organischer Lösung, bevorzugt einen Feststoffgehalt von 50 bis 80 Gew.-% aufweisend, oder aus der Schmelze erfolgen, wobei im Falle der Verwendung organischer Lösungen der erfindungsgemäßen Polymerisate das Lösungsmittel nach dem Beschichten der Trägermaterialoberfläche im allgemeinen mittels Wärme ausgetrieben wird. Vorzugsweise wird aus der Schmelze, bei Temperaturen von 80 bis 120°C, appliziert. Die Applikation kann in an sich bekannter Weise durch Streichen, Verdüsen, Walzen, Rakeln oder Gießen erfolgen. Aufgrund der erhöhten thermischen Stabilität der Bindung des Phenongerüsts an das Polymerrückgrat in den erfindungsgemäßen Polymerisaten, mindert auch eine Verarbeitung der erfindungsgemäßen Haftklebstoffe bei Temperaturen um 150°C deren Reaktivität gegenüber UV-Strahlung nur unwesentlich.

Die Bestrahlung mit ultraviolettem Licht kann unmittelbar nach dem Auftragen, nach der Entfernung des Lösungsmittels (Applikation aus der Lösung) oder nach Durchlaufen einer Heiz-, Temper- und/oder Kühlstrecke (insbesondere bei Applikation aus der Schmelze) erfolgen. Zum Bestrahlen können handelsübliche UV-Strahler, die vorzugsweise in einem Wellenlängenbereich von 250 bis 400 nm Strahlung emittieren, eingesetzt werden. Geeignet sind beispielsweise Quecksilbermitteldruckstrahler mit einer Strahlungsleistung von 80 bis 120 W/cm, wie sie z.B. in "Sources and Applications of Ultraviolett Radiation", R. Philips, Academic Press, London 1983, beschrieben werden. Die Bestrahlungsdauer richtet sich nach der Dicke der Beschichtung, dem UV-Emissionsspektrum und der Strahlungsleistung der verwendeten Strahlungsquelle sowie den jeweils einpolymerisierten Phenonen I. Sie kann jedoch in Vorversuchen leicht ermittelt werden. Die Bestrahlung erfordert keine Gegenwart von Schutzgas.

Beispiele

Beispiel 1

Herstellung verschiedener erfindungsgemäßer Phenone I

Für alle nachfolgenden Phenone I wurde die Struktur durch $^{1}H$-NMR-, $^{13}C$-NMR-, IR- und Massenspektroskopie sowie partiell durch unabhängige Synthesen bestätigt.

a) 3-Acrylamidomethyl-4-methoxy-propiophenon

Ein Gemisch aus 400 g Schwefelsäure, 200 g Essigsäure, 82,0 g (0,5 Mol) 4-Methoxy-propiophenon, 0,2 g Di-tert.-butyl-p-kresol und 55,5 g (0,55 Mol) N-Methylolacrylamid wurde 4 h bei 20°C umgesetzt und anschließend mit Eis hydrolysiert. Die dabei fest anfallende Phase wurde durch Filtration abgetrennt, in 1 l Methylenchlorid aufgenommen und über eine mit Aluminiumoxid bepackte Säule chromatographisch gereinigt.

Im Anschluß wurde das Lösungsmittel bei 30°C und 20 mbar entfernt und der dabei anfallende Rückstand (92 g, Rohausbeute = 75 % d. Theorie) mit Petrolether ausgerührt.

Dabei fiel das Produkt in analysenreiner Form an.

Reinausbeute: 82 g (66,8 % d. Theorie)
Smp.: 122 bis 124°C.

| | | | | |
|---|---|---|---|---|
| Elementaranalyse | Theorie; | C 67,99 | H 6,93 | N 5,66 |
| | Exp.: | C 68,2 | H 6,9 | N 5,4 |

b) 3-Acrylamidomethyl-4-methoxy-benzophenon

Ein Gemisch aus 1000 g Schwefelsäure, 175,6 g (0,825 Mol) 4-Methoxybenzophenon, 0,5 g 2,5-Di-tert.-butyl-p-kresol und 86 g (0,851 Mol) N-Methylolacrylamid wurde 5 h bei 25°C umgesetzt und anschließend auf Eis hydrolysiert.

Die dabei anfallende organische Phase wurde in Methylenchlorid aufgenommen und mehrfach mit Wasser gewaschen. Im Anschluß wurde das Lösungsmittel bei 30°C und 20 mbar entfernt und der dabei erhaltene Rückstand (238 g, Rohausbeute 98 % d. Theorie) in 1000 ml einer Mischung aus Ethylacetat und Cyclohexan im Volumenverhältnis 5 : 1 umkristallisiert.

Reinausbeute: 174 g (71,6 % d. Theorie)
Smp.: 117 - 119°C

| | | | | |
|---|---|---|---|---|
| Elementaranalyse | Theorie: | C 73,20 | H 5,80 | N 4,74 |
| | Exp.: | C 73,10 | H 5,7 | N 4,8 |

Durch analoge Umsetzungen wurden die Verbindungen c) bis g) der Tabelle 1 erhalten.

Tabelle 1

| Ausgangsverbindungen | Reaktionsbedingungen | Produkt |
|---|---|---|
| c) 4-Hydroxybenzophenon<br>N-Methylolacrylamid | Schwefelsäure/Essigsäure | 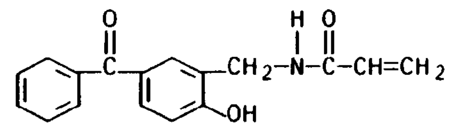<br>Schmp.: 160 bis 161°C<br>Th.: C 72,58 H 5,37 N 4,98<br>Exp.: C 72,2 H 5,5 N 4,9 |
| d) 6-Methoxy-1-tetralon<br>N-Methylolacrylamid | Schwefelsäure/Essigsäure<br>(Gewichtsverh. 2:1)<br>20°C, 5h | 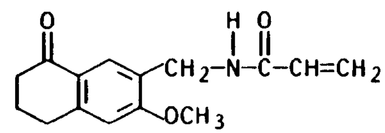 15 Gew.-%<br>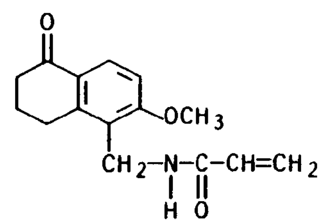 85 Gew.-%<br>Schmp.: 158 bis 163°C (des Gemisches)<br>Th.: C 69,48 H 6,61 N 5,40<br>Exp.: C 69,1 H 6,7 N 5,5 |

Tabelle 1 - Fortsetzung -

| Ausgangsverbindungen | Reaktionsbedingungen | Produkt |
|---|---|---|
| e) p-Methoxyacetophenon<br>N-Methylolacrylamid | Schwefelsäure/Essigsäure<br>(Gewichtsverh. 2:1)<br>15 bis 20°C, 4h | 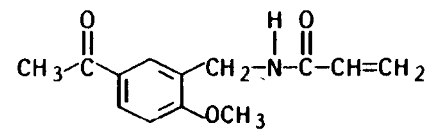<br>Schmp.: 104 bis 106°C<br>Th.: C 66,94 H 6,48 N 6,00<br>Exp.: C 67,0 H 6,5 N 5,9 |
| f) p-Methyl-benzophenon<br>N-Methylolacrylamid | Schwefelsäure<br>40 bis 50°C, 7h | 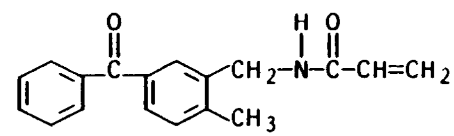<br>Schmp.: 149 bis 151°C<br>Th.: C 77,40 H 6,13 N 5,01<br>Exp.: C 77,0 H 6,3 N 4,9 |
| g) p-Methoxy-benzophenon<br>N-Methylolmethacrylamid | Methansulfonsäure<br>20 bis 25°, 5h | 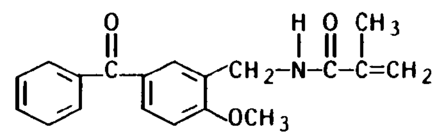<br>Schmp.: 88 bis 90°C<br>Th.: C 73,77 H 6,19 N 4,53<br>Exp.: C 73,7 H 6,3 N 4,5 |

Die Beispiele h) und i) belegen die Möglichkeit der nachträglichen Funktionalisierung primär erhaltener

Phenone I.

h) 2-Acrylamidomethyl-4-benzoyl-phenoxyessigsäure

Ein Gemisch aus 10,42 g (0,075 Mol) Bromessigsäure, 60 ml Wasser, 3 g (0,075 Mol) Natriumhydroxid und 13,8 g (0,049 Mol) 3-Acrylamidomethyl-4-hydroxy-benzophenon wurde bei 10 bis 15°C mit einer Lösung von 1,97 g (0,049 Mol) Natriumhydroxid in 20 ml Wasser versetzt und anschließend 1,5 h bei 80°C gehalten.

Nach Abkühlen auf 20°C wurde mit 20 ml 2N-Salzsäure angesäuert, das abgeschiedene Produkt mit Methylenchlorid aufgenommen und die Methylenchloridlösung bei Raumtemperatur eingedampft. Der harzartige Rückstand (17,20 g) wurde in 85 ml Ethylacetat gelöst und 24 h bei 25°C gehalten. Die dabei ausfallenden Kristalle wurden durch Filtration abgetrennt.

| | |
|---|---|
| Reinausbeute: | 9,51 g |
| Smp.: | 154 bis 156°C |

| | | | | |
|---|---|---|---|---|
| Elementaranalyse | Theorie: | C 67,25 | H 5,05 | N 4,13 |
| | Exp.: | C 67,1 | H 5,1 | N 4,0 |

i) N-Cyclohexylcarbamidsäure-(2-acrylamidomethyl-4-benzoyl-phenyl)-ester

Ein Gemisch aus 30 g (0,107 Mol) 3-Acrylamidomethyl-4-hydroxy-benzophenon, 13,5 g (0,108 Mol) Cyclohexylisocyanat, 0,2 g 2,6-Di-tert.-butyl-p-kresol, 1,5 g Triethylamin und 180 ml Aceton wurde 3 h unter Rückfluß gehalten und anschließend sich selbst überlassen. Die dabei gebildeten Kristalle wurden abfiltriert und so 26,3 g des Zielproduktes mit einem Smp. von 142 bis 144°C erhalten. Durch Eindampfen der Mutterlauge wurden weitere 13,5 g des gewünschten Carbamats gewonnen.

Beispiel 2

Polymerisate, die erfindungsgemäße Phenone I einpolymerisiert enthalten und nach UV-Bestrahlung als Haftklebstoffe mit erhöhter Scherstandfestigkeit geeignet sind

P1: 100 g einer Monomerenmischung 1 aus

| | |
|---|---|
| 840 g | iso-Amylacrylat |
| 130 g | Methylacrylat |
| 30 g | Acrylsäure und |
| 0,65 g | 3-Acrylamidomethyl-4-methoxy-benzophenon |

wurde in 200 g Essigsäureethylester (Lösungsmittel) in Anwesenheit von 4 g 2,2'-Azobis(methylisobutyrat) (Polymerisationsinitiator) auf die Polymerisationstemperatur von 77°C erhitzt und anschließend im Verlauf von 3 h unter Aufrechterhaltung der Polymerisationstemperatur mit dem Rest der Monomerenmischung 1 sowie dazu parallel im Verlauf von 4 h mit einer Lösung von 10 g 2,2'-Azobis(methylisobutyrat) in 50 g Essigsäureethylester versetzt. Danach wurde noch bei Temperaturen von 75 bis 85°C 4 h nachpolymerisiert und schließlich das Lösungsmittel destillativ abgetrennt.

Es wurde ein bei Raumtemperatur fließfähiges Polymerisat erhalten, das in THF (25°C) einen K-Wert von 42 aufwies.

P2: Wie P1, jedoch wurde anstelle der Monomerenmischung 1 eine Monomerenmischung 2 eingesetzt, bestehend aus:

| | |
|---|---|
| 545 g | n-Butylacrylat |
| 300 g | 2-Ethylhexylacrylat |
| 130 g | N-Vinylpyrrolidon |
| 35 g | Acrylsäure und |
| 0,55 g | 3-Acrylamidomethyl-4-methoxybenzophenon |

Es wurde ein bei Raumtemperatur fließfähiges Polymerisat erhalten, das in THF (25°C) einen K-Wert von 48 aufwies.

Beispiel 3

Prüfung der Haftklebeeigenschaften der Polymerisate P1 und P2, aus Beispiel 2, nach UV-Bestrahlung

a) Herstellung der Prüfstreifen

Zur Herstellung der Prüfstreifen wurden die Polymerisate P1 und P2 aus ihrer Schmelze in einer Schichtdicke von 25 g/m² auf eine Polyesterfolie als Träger aufgetragen.

Anschließend wurde die Polyesterfolie mit einer Geschwindigkeit von 20 m/min im Abstand von 10 cm unter zwei hintereinander (im Abstand von 11 cm) angeordneten Quecksilbermitteldruckstrahlern (80 W/cm) durchgeführt. Aus der so erhaltenen selbstklebenden Folie wurden Streifen von 2 cm Breite und 5 cm Länge ausgeschnitten.

b) Prüfung der Scherstandfestigkeit

Die Prüfstreifen (a) wurden auf einer Länge von 2,5 cm, unter Anwendung eines Gewichtes der Masse 2,5 kg, auf ein chromiertes Stahlblech (V2A) aufgerollt und 24 h unter Normalbedingungen gelagert. Anschließend wurde das nicht behaftete Ende des Stahlblechs zwischen 2 Klemmbacken befestigt und das gegenüberliegende überstehende Klebeband frei hängend bei Temperaturen von 25° mit einem Gewicht der Masse 2 kg und bei 50°C mit einem Gewicht der Masse 1 kg belastet. Maßstab für die Scherstandfestigkeit ist die Zeitdauer bis zum Aufbrechen des Klebstoffilms. Zum Vergleich wurde die Prüfung mit selbstklebenden Folien wiederholt, die nicht bestrahlt worden waren. Die Ergebnisse zeigt Tabelle 2.

c) Prüfung der Schälfestigkeit

Zur Bestimmung der Schälfestigkeit der Prüfstreifen (a) auf der Oberfläche eines Substrates, wurden diese auf einer Länge von 2,5 cm, unter Anwendung eines Gewichtes der Masse 2,5 kg, auf ein chromiertes Stahlblech (V2A) aufgerollt. 24 h danach wurde die Kraft bestimmt, um die Prüfstreifen in einer Zugdehnungsprüfapparatur bei einem Abschälwinkel von 180°C mit einer Geschwindigkeit von 300 mm/min rückwärts abzuziehen. Die Ergebnisse enthält ebenfalls Tabelle 2.

Tabelle 2

| | Scherstandfestigkeit [h] | | Schälfestigkeit [N/cm] |
|---|---|---|---|
| | 25°C | 50°C | |
| P1 (bestrahlt) | >24 | >24 | 4,3 |
| P1 | < 1 | < 1 | - |
| P2 (bestrahlt) | >24 | >24 | 8,4 |
| P2 | - | < 1 | - |

Beispiel 4

Prüfung der Temperaturbeständigkeit des Polymerisates P1 und zweier Vergleichspolymerisate P1' , P1".

Die Polymerisate wurden in Luftsauerstoffatmosphäre 12 h einer Temperatur von 150°C ausgesetzt. Anschließend wurde auf vernetzte Anteile und Farbveränderungen untersucht. Die Polymerisate P1' und P1" unterscheiden sich von P1 nur dadurch, daß anstelle des entsprechenden Phenons I die nachfolgenden Phenonmonomere, aus DE-A 38 20 464, eingesetzt wurden:

P' :

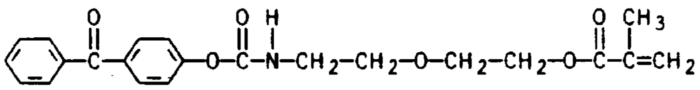

Pl'':

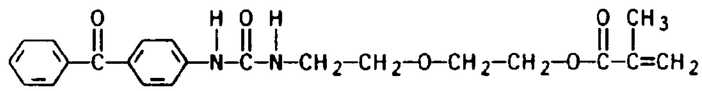

Die Ergebnisse zeigt Tabelle 3.

Tabelle 3

| | Vernetzte Anteile | Farbveränderung |
|---|---|---|
| Pl | keine Gelkörper | keine |
| Pl' | Gelkörper | keine |
| Pl'' | keine Gelkörper | gelbbraun |

## Patentansprüche

1. Ungesättigte Verbindungen der allgemeinen Formel I

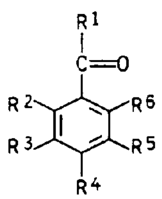

(I),

in der die Variablen folgende Bedeutung haben:

$R^1$ eine 1 bis 4 C-Atome enthaltende Alkylgruppe, einen Cyclopropyl-, -pentyl- oder -hexylrest, die Phenylgruppe oder eine Phenylgruppe, deren Wasserstoffatome teilweise oder vollständig durch Reste $R^4$ ersetzt sind, wobei nicht mehr als zwei Substituenten $R^4$ identisch sind, sowie $R^1$ gemeinsam mit $R^2$ oder gemeinsam mit $R^6$ eine $-CH_2-CH_2-$ oder $-CH_2-CH_2-CH_2-$Brücke,

$R^4$ eine 1 bis 24 C-Atome enthaltende Alkylgruppe,

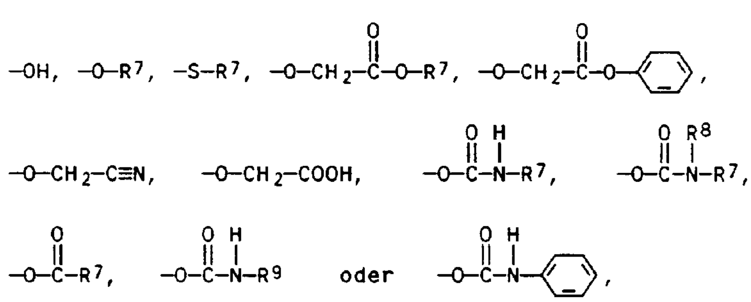

oder

$R^2$ oder $R^6$ unabhängig voneinander Wasserstoff, einen der Reste $R^4$ mit der Ausnahme der OH - Gruppe, oder für den Fall, daß $R^1$ eine Arylgruppe ist, eine direkte Bindung an $R^1$ in ortho-Stellung zur Carbonylgruppe, und

$R^3$, $R^5$ Wasserstoff, einen der Reste $R^4$ oder eine Gruppe der allgemeinen Formel II

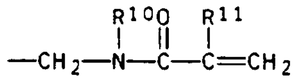 (II),

wobei

$R^7$, $R^8$ eine 1 bis 4 C-Atome enthaltende Alkylgruppe,

$R^9$ eine 5 oder 6 C-Atome enthaltende Cycloalkylgruppe,

$R^{10}$ Wasserstoff oder eine 1 bis 4 C-Atome enthaltende Alkylgruppe und

$R^{11}$ Wasserstoff oder eine 1 bis 4 C-Atome enthaltende Alkylgruppe bedeuten,

mit der Maßgabe, daß entweder $R^3$ oder $R^5$ eine Gruppe der allgemeinen Formel II ist.

2. Verbindungen nach Anspruch 1, die als $R^4$ eine 1 bis 4 C-Atome aufweisende Alkoxygruppe enthalten.

3. Verbindungen nach Anspruch 2, die als $R^{11}$ Wasserstoff oder die Methylgruppe, als $R^1$ die Phenylgruppe sowie als $R^{10}$, $R^2$, $R^3$ und $R^6$ jeweils Wasserstoff aufweisen.

4. Polymerisate, die Monomere gemäß den Ansprüchen 1 bis 3 einpolymerisiert enthalten.

5. Copolymerisate, die in polymerisierter Form aus
   a) 0,25 bis 5 Gew.-% wenigstens eines Monomeren gemäp den Ansprüchen 1 bis 4 und
   b) 95 bis 99,75 Gew.-% wenigstens eines copolymerisierbaren monoethylenisch ungesättigten Monomeren

   aufgebaut sind, eine Glasübergangstemperatur von -45 bis 0°C und in Tetrahydrofuran bei 25°C einen K-Wert von 20 bis 70 aufweisen.

6. Copolymerisate nach Anspruch 5, die eine Glasübergangstemperatur von -30 bis -10°C und in Tetrahydrofuran bei 25°C einen K-Wert von 30 bis 55 aufweisen.

7. Verfahren zur Herstellung von Monomeren gemäß den Ansprüchen 1 bis 3, dadurch gekennzeichnet, daß man eine Verbindung der allgemeinen Formel V

   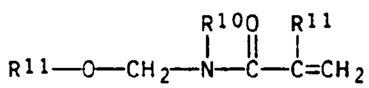 (V),

   mit einer Verbindung der allgemeinen Formel VI

   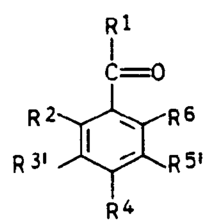 (VI),

   in der einer der Reste $R^{3'}$ oder $R^{5'}$ Wasserstoff und der andere ein Rest $R^4$ ist, umsetzt.

8. Verwendung von Copolymerisaten gemäß den Ansprüchen 4 bis 6 als Basis für Haftklebstoffe.

9. Selbstklebende Artikel, die aus einem Trägermaterial und einem Haftklebstoff auf der Basis von Copolymerisaten gemäß den Ansprüchen 4 bis 6 bestehen.

10. Verfahren zur Herstellung selbstklebender Artikel nach Anspruch 9, dadurch gekennzeichnet, daß das Trägermaterial mit einem Copolymerisat gemäß den Ansprüchen 4 bis 6 aus
organischer Lösung oder aus der Schmelze beschichtet und danach mit ultravioletter Strahlung bestrahlt

wird.

## Claims

1. An unsaturated compound of the formula I

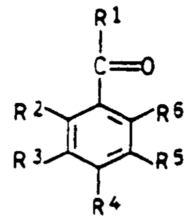

(I)

where
$R^1$ is alkyl of 1 to 4 carbon atoms, cyclopropyl, cyclopentyl, cyclohexyl, phenyl or phenyl in which some or all of the hydrogen atoms have been replaced by radicals $R^4$, not more than two substituents $R^4$ being identical, or $R^1$ together with $R^2$ or together with $R^6$ forms a $-CH_2-CH_2-$ or $-CH_2-CH_2-CH_2-$ bridge,
$R^4$ is alkyl of 1 to 24 carbon atoms,

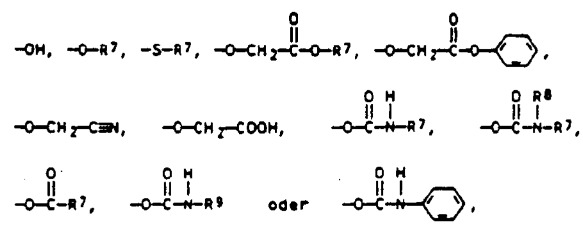

oder

or $R^2$ or $R^6$, independently of one another, is each hydrogen, one of the radicals $R^4$, with the exception of OH, or, where $R^1$ is aryl, is a direct bond to $R^1$ in the ortho-position with respect to the carbonyl group, and
$R^3$ and $R^5$are each hydrogen, one of the radicals $R^4$ or a group of the formula II

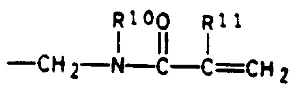

(II)

where
$R^7$ and $R^8$ are each alkyl of 1 to 4 carbon atoms,
$R^9$ is cycloalkyl of 5 or 6 carbon atoms,
$R^{10}$ is hydrogen or alkyl of 1 to 4 carbon atoms and
$R^{11}$ is hydrogen or alkyl of 1 to 4 carbon atoms,
with the proviso that either $R^3$ or $R^5$ is a group of the formula II.

2. A compound as claimed in claim 1, which contains alkoxy of 1 to 4 carbon atoms as $R^4$.

3. A compound as claimed in claim 2, which has hydrogen or methyl as $R^{11}$, phenyl as $R^1$ and hydrogen as $R^{10}$, $R^2$, $R^3$ and $R^6$.

4. A polymer which contains monomers as claimed in claim 1 or 2 or 3 as polymerized units.

5. A copolymer which, in polymerized form, is composed of
   a) from 0.25 to 5% by weight of one or more monomers as claimed in claim 1 or 2 or 3 or 4 and
   b) from 95 to 99.75% by weight of one or more copolymerizable monoethylenically unsaturated monomers,
has a glass transition temperature of from -45 to 0°C and has a K value of from 20 to 70 in tetrahydrofuran at 25°C.

6. A copolymer as claimed in claim 5, which has a glass transition temperature of from -30 to -10°C and has a K value of from 30 to 55 in tetrahydrofuran at 25°C.

7. A process for the preparation of a monomer as claimed in claim 1 or 2 or 3, wherein a compound of the formula V

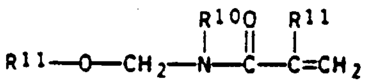

(V)

is reacted with a compound of the formula VI

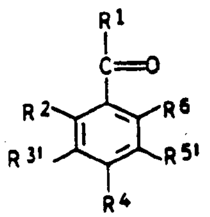

(VI)

where one of the radicals $R^{3'}$ or $R^{5'}$ is hydrogen and the other is a radical $R^4$.

8. Use of a copolymer as claimed in claim 4 or 5 or 6 as a basis for contact adhesives.

9. A self-adhesive article which consists of a substrate and a contact adhesive based on a copolymer as claimed in claim 4 or 5 or 6.

10. A process for the production of a self-adhesive article as claimed in claim 9, wherein the substrate is coated with a copolymer as claimed in claim 4 or 5 or 6 from organic solution or from the melt and then exposed to ultraviolet radiation.

## Revendications

1. Composés insaturés de la formule générale I

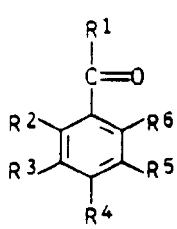

(I),

dans laquelle les variables ont les significations suivantes :

$R^1$ représente un radical alkyle contenant de 1 4 atomes de carbone, un radical cyclopropyle, cyclopentyle ou cyclohexyle, le radical phényle ou un radical phényle dont les atomes d'hydrogène sont partiellement ou totalement remplacés par des restes $R^4$ où pas plus de deux substituants $R^4$ sont identiques, come aussi $R^1$ ensemble avec $R^2$ , ou ensemble avec $R^6$, forme un pont $-CH_2-CH_2-$ ou un pont $-CH_2-CH_2-CH_2-$,

$R^4$ représente un radical alkyle contenant de 1 à 24 atomes de carbone,

$-OH$, $-O-R^7$, $-S-R^7$, 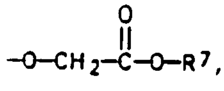, 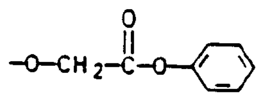,

$-O-CH_2-C{\equiv}N$, $-O-CH_2-COOH$, 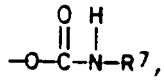, 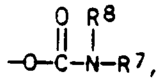,

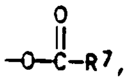, 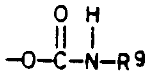 ou 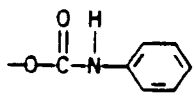,

$R^2$ et $R^6$ représentent, chacun indépendamment l'un de l'autre, un atome d'hydrogène, l'un des substituants $R^4$ à l'exception du radical OH, ou, au cas où $R^1$ représente un radical aryle, une liaison directe à $R^1$ en position ortho par rapport au radical carbonyle

$R^3$, $R^4$ représentent chacun un atome d'hydrogène, l'un des substituants $R^4$, ou un groupe de la formule générale II

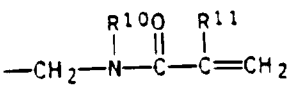 (II),

dans laquelle

$R^7$, $R^8$ représentent chacun un radical alkyle contenant de 1 à 4 atomes de carbone,

$R^9$ représente un radical cycloalkyle contenant 5 ou 6 atomes de carbone,

$R^{10}$ représente un atome d'hydrogène ou un radical alkyle contenant de 1 à 4 atomes de carbone et

$R^{11}$ représente un atome d'hydrogène ou un radical alkyle qui contient de 1 à 4 atomes de carbone,

avec la condition que $R^3$ ou $R^5$ représente un radical de la formule générale II.

2. Composés suivant la revendication 1, caractérisés en ce qu'ils contiennent, à titre de $R^4$, un radical alcoxy présentant de 1 à 4 atomes de carbone.

3. Composés suivant la revendication 2, caractérisés en ce qu'à titre de $R^{11}$, ils contiennent de l'hydrogène ou le radical méthyle, qu'à titre de $R^1$, ils contiennent le radical phényle et qu'à titre de $R^{10}$, $R^2$, $R^3$ et $R^6$ ils contiennent à chaque fois de l'hydrogène.

4. Polymères qui contiennent des monomères suivant l'une quelconque des revendications 1 à 3, incorporés par polymérisation.

5. Copolymères qui, sous forme polymérisée, sont constitués de
   a) 0,25 à 5% en poids d'au moins un monomère suivant l'une quelconque des revendications 1 à 4 et
   b) 95 à 99,75% en poids d'au moins un monomère monoéthyléniquement insaturés, copolymérisable,
   possèdent une température de transition vitreuse de -45 à 0°C et une valeur K de 20 à 70 dans le tétrahydrofuranne à 25°C.

6. Copolymères suivant la revendication 5, qui possèdent une température de transition vitreuse de -30 à -10°C et présentent une valeur K de 30 à 55 dans le tétrahydrofuranne à 25°C.

7. Procédé de préparation de monomères suivant l'une quelconque des revendications 1 à 3, caractérisé en ce que l'on fait réagir un composé de la formule générale V

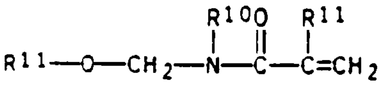

(V),

avec un composé de la formule générale VI

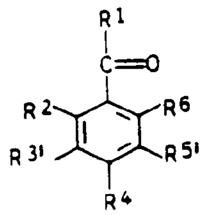

(VI),

dans laquelle l'un des symboles $R^{3'}$ et $R^{5'}$ représente un atome d'hydrogène et l'autre représente un reste $R^4$.

**8.** Utilisation de copolymères suivant les revendications 4 à 6 à titre de base pour des colles de contact.

**9.** Articles autocollants, qui sont constitués d'un matériau de support et d'une colle de contact à base de copolymère suivant l'une quelconque des revendications 4 à 6.

**10.** Procédé de préparation d'articles autocollants suivant la revendication 9, caractérisé en ce que l'on revêt le matériau de support d'un copolymère suivant l'une quelconque des revendications 4 à 6, à partir d'une solution organique ou à partir de la masse fondue et on soumet ensuite l'article à un rayonnement ultraviolet.